(19) Europäisches Patentamt
European Patent Office
Office européen des brevets

(11) Veröffentlichungsnummer: **0 256 400 B1**

# (12) EUROPÄISCHE PATENTSCHRIFT

(45) Veröffentlichungstag der Patentschrift: **23.09.92**

(51) Int. Cl.$^{5}$: **C07H 19/073**, A61K 31/70

(21) Anmeldenummer: **87111133.2**

(22) Anmeldetag: **31.07.87**

Die Akte enthält technische Angaben, die nach dem Eingang der Anmeldung eingereicht wurden und die nicht in dieser Patentschrift enthalten sind.

## (54) Pyrimidinderivate, ihre Herstellung und Medikamente die diese Derivate enthalten.

(30) Priorität: **12.08.86 GB 8619630**
**07.05.87 GB 8710775**

(43) Veröffentlichungstag der Anmeldung:
**24.02.88 Patentblatt 88/08**

(45) Bekanntmachung des Hinweises auf die Patenterteilung:
**23.09.92 Patentblatt 92/39**

(84) Benannte Vertragsstaaten:
**AT BE CH DE ES FR GB GR IT LI LU NL SE**

(56) Entgegenhaltungen:
**US-A- 4 000 260**

**JOURNAL OF MEDICINAL CHEMISTRY, Band 24, Nr. 3, 1981, Seite 350-352, American Chemical Society, Columbus, Ohio, US; R.D. ELLIOTT: "Nucleosides containing chemically reactive groups"**

(73) Patentinhaber: **F. HOFFMANN-LA ROCHE AG**
**Postfach 3255**
**CH-4002 Basel(CH)**

(72) Erfinder: **Lambert, Robert Wilson**
**33 New Road Digswell**
**Welwyn Herts(GB)**
Erfinder: **Martin, Joseph Armstrong**
**10 The Chownes West Common**
**Harpenden Herts(GB)**
Erfinder: **Thomas, Gareth John**
**2B Woodland Way Oaklands**
**Welwyn Herts(GB)**

(74) Vertreter: **Lederer, Franz, Dr. et al**
**Lederer, Keller & Riederer Patentanwälte**
**Lucile-Grahn-Strasse 22**
**W-8000 München 80(DE)**

**EP 0 256 400 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft Pyrimidinderivate, ein Verfahren zu deren Herstellung und Medikamente, die diese Derivate enthalten.

Die erfindungsgemässen Pyrimidinderivate besitzen die allgemeine Formel

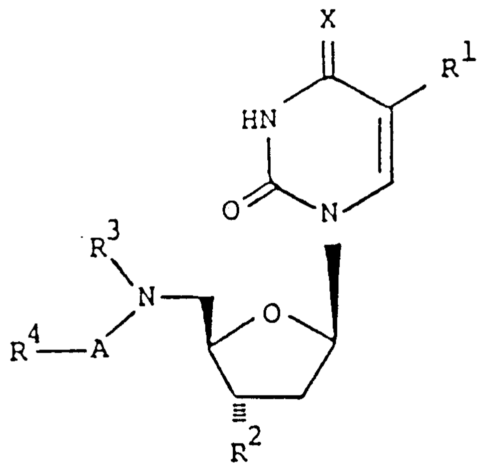

I

worin A $C_{1-8}$-Alkylen, $R^1$ Halogen, $C_{1-4}$-Alkyl oder Halo-($C_{1-4}$-alkyl), $R^2$ Wasserstoff, Hydroxy oder Acyloxy, $R^3$ Wasserstoff oder $C_{1-4}$-Alkyl, $R^4$ Aryl oder Aryloxy und X Sauerstoff oder NH bedeutet, und Tautomere davon.

In der US Patentschrift 4000260 sind 5'-amino-2',5'-dideoxyuridine beschrieben. Im Gegensatz zu den Verbindungen der Formel I der vorliegenden Erfindung enthalten die vorbekannten Verbindungen keine substituierte Aminogruppe in 5'-Stellung.

In Antiviral Research, 2 (1982) 319-330 werden N-acylierte Derivate des Inhibitors der Thymidinkinase AldU offenbart, die - vergleiche das N-benzoylierte Derivat Nr. 22 - dem N-unsubstituierten AldU sogar noch überlegen sind.

Der hier verwendete Ausdruck "$C_{1-4}$-Alkyl" bedeutet eine geradkettige oder verzeigte Alkylgruppe mit 1-4 C-Atomen, wie Methyl, Aethyl, Propyl, Isopropyl, Butyl oder t-Butyl. "Halo-($C_{1-4}$-alkyl)"bedeutet eine wie vorstehend definierte Alkylgruppe, die ein oder mehrere Halogenatome trägt, beispielsweise Trifluormethyl oder 2-Chloräthyl. "$C_{1-8}$-Alkylen" bedeutet eine geradkettige oder verzweigte Alkylengruppe mit 1-8 C-Atomen, wie $-CH_2-$, $-CH_2CH_2-$, $-CH(CH_3)-$, $-CH_2CH_2CH_2-$, $-CH_2-CH(CH_3)-$ oder $-CH_2CH_2CH_2CH_2-$. Die Acyloxygruppe kann sich von einer aliphatischen, cycloaliphatischen, araliphatischen oder aromatischen Carbonsäure ableiten, wobei Beispiele solcher Säuren Ameisensäure, Essigsäure, Propionsäure, Buttersäure, Cyclopentylpropionsäure, Phenylessigsäure und Benzoesäure sind. Bevorzugte Acyloxygruppen sind $C_{1-4}$-Alkanoyloxygruppen. "Aryl" bedeutet eine unsubstituierte Phenylgruppe oder eine Phenylgruppe, die einen oder mehrere Substituenten aus der Reihe Halogen, Hydroxy, $C_{1-4}$-Alkyl, $C_{1-4}$-Alkoxy, Trifluormethyl, Nitro und Phenyl enthält. Beispiele solcher substituierten Phenylgruppen sind 2-Bromphenyl, 4-Chlorphenyl, 2,3-Dichlorphenyl, 2,6-Dichlorphenyl, 2-Methylphenyl und 2,6-Dimethylphenyl. "Aryloxy" bedeutet eine wie vorstehend definierte Arylgruppe, die über ein Sauerstoffatom gebunden ist. Beispiele von Aryloxygruppen sind Phenoxy, 4-Chlorphenoxy, 2,4-Dichlorphenoxy und 2,6-Dichlorphenoxy. "Halogen" bedeutet Fluor, Chlor, Brom und Jod.

Je nach der Bedeutung von A in Formel I können die Verbindungen der Formel I und deren Tautomere als Diastereomere vorliegen. Die vorliegende Erfindung umfasst nicht nur die einzelnen Diastereomere sondern auch Gemische davon.

In Formel I ist A vorzugsweise $C_{1-4}$-Alkylen, insbesondere $-CH_2-$, $-CH_2CH_2-$, $-CH(CH_3)-$ oder $-CH_2-CH(CH_3)-$. $R^1$ ist vorzugsweise $C_{1-4}$-Alkyl, insbesondere Aethyl. $R^2$ ist vorzugsweise Hydroxy. $R^3$ ist vorzugsweise Wasserstoff oder Methyl. $R^4$ ist vorzugsweise Dihalophenyl, insbesondere 2,3- oder 2,6-Dichlorphenyl. X ist vorzugsweise Sauerstoff.

Besonders bevorzugte Verbindungen der Erfindung sind diejenigen, in denen A $-CH_2-$, $-CH_2CH_2-$, $-CH(CH_3)$ -oder $-CH_2-CH(CH_3)-$, $R^1$ is Aethyl, $R^2$ Hydroxy, $R^3$ Wasserstoff oder Methyl, $R^4$ 2,3- oder 2,6-Dichlorphenyl und X Sauerstoff sind.

Besonders bevorzugte Verbindungen sind:

5'-(2,6-Dichlorbenzylamino)-2',5'-dideoxy-5-äthyluridin,

5'-[2-(2,6-Dichlorphenyl)äthylamino]-2',5'-dideoxy-5-eähyluridin und
5'-(2,3-Dichlorbenzylamino)-2',5'-dideoxy-5-äthyluridin.

Beispiele anderer interessierender Verbindungen der vorliegenden Erfindung sind:
5'-(4-Chlorbenzylamino)-5'-deoxythymidin,
5'-(2-Brombenzylamino)-2',5'-dideoxy-5-äthyluridin,
5'-[2-(2-Bromophenyl)äthylamino]-2',5'-dideoxy-5-äthyluridin,
2',5'-Dideoxy-5-äthyl-5'-[2-(2,6-dimethylphenyl)äthylamino]uridin,
5'-(4-Chlorbenzylamino)-2',5'-dideoxy-5-äthyluridin,
2',5'-Dideoxy-5-äthyl-5'-[1(R)-phenyläthylamino]uridin,
2',5'-Dideoxy-5-äthyl-5'-[1(S)-phenyläthylamino]uridin,
2',5'-Dideoxy-5-äthyl-5'-(N-methylbenzylamino)uridin,
2',5'-Dideoxy-5-äthyl-5'-(phenyläthylamino)uridin,
2',5'-Dideoxy-5-äthyl-5'-[2-(2-methylphenyl)äthylamino]uridin,
2',5'-Dideoxy-5-äthyl-5'-(N-methylphenyläthylamino)uridin,
5'-[1(S)-Benzyläthylamino]-2',5'-dideoxy-5-äthyluridin,
5'-Benzylamino-2',5'-dideoxy-5-äthyluridin,
2',5'-Dideoxy-5-äthyl-5'-(2-methylbenzylamino)uridin und
5'-Benzylamino-5'-deoxythymidin.

2',5'-Dideoxy-5-äthyl-5' -[2(RS)-(2,4-dichlorphenoxy)propylamino]uridin ist ein Beispiel einer weiteren interessierenden Verbindung der vorliegenden Erfindung.

Die Verbindungen der obigen Formel I und deren Tautomere können erfindungsgemäss dadurch hergestellt werden, dass man

a) eine Verbindung der Formel

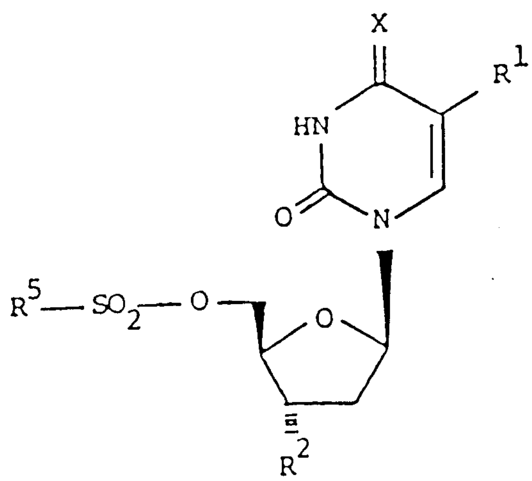

II

worin $R^1$, $R^2$ und X die obige Bedeutung haben und $R^5$ $C_{1-4}$-Alkyl oder Aryl ist, oder ein Tautomer davon mit einer Verbindung der Formel

$R^3$-NH-A-$R^4$ III

worin A, $R^3$ und $R^4$ die obige Bedeutung haben, bei erhöhter Temperatur umsetzt oder

b) zur Herstellung einer Verbindung der Formel I, in der X O ist oder eines Tautomeren davon, die Gruppe $R^4$-A in eine Verbindung der Formel

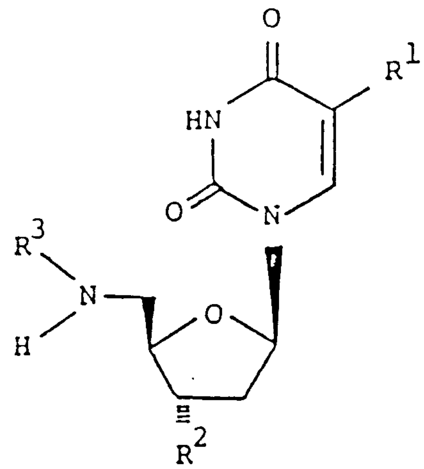

IV

worin $R^1$, $R^2$ und $R^3$ die obige Bedeutung haben,
oder ein Tautomer davon durch reduktive Alkylierung einführt.

Die Umsetzung gemäss Ausführungsform a) des Verfahrens wird zweckmässig in Gegenwart eines inerten organischen Lösungsmittels wie Dimethylformamid (DMF), Acetonitril oder Dimethylsulfoxid (DMSO) vorgenommen. Das bevorzugte Lösungsmittel ist DMF und in diesem Falle wird das Reaktionsgemisch zweckmässig auf etwa 80°C erwärmt. Alternativ kann ein Ueberschuss eines Amins der Formel III angewandt werden und dieses als Lösungsmittel dienen. Die Gruppe $R^5$ in Formel II ist vorzugsweise Aryl, insbesondere p-Tolyl.

Die reduktive Alkylierung gemäss Ausführungsform b) des Verfahrens kann in an sich bekannter Weise ausgeführt werden. Beispielsweise wird eine Verbindung der Formel IV oder ein Tautomer davon mit einem geeigneten Aldehyd oder Keton umgesetzt und die erhaltene Schiffsche Base dann katalytisch in situ hydriert, wobei die gewünschte Verbindung der Formel I oder deren Tautomer erhalten wird. Die reduktive Alkylierung wird zweckmässig in einem inerten organischen Lösungsmittel, z.B. einem Alkanol wie Methanol oder Aethanol durchgeführt. Die katalytische Hydrierung kann unter konventionellen Bedingungen, z.B. mit einem Edelmetallkatalysator wie Palladium- oder Platin-Katalysatoren, die auf einem inerten Träger aufgebracht sein können, durchgeführt werden. Palladium auf Kohle (Pd/C) ist der bevorzugte Katalysator. Zweckmässig wird die katalytische Hydrierung bei etwa Raumtemperatur und unter Atmosphärendruck durchgeführt. Alternativ kann eine Verbindung der Formel IV oder ein Tautomer davon mit einem geeigneten Aldehyd oder Keton in Gegenwart von Natriumcyanborhydrid in einem geeigneten Lösungsmittel, z.B. einem wässrigen Alkanol wie wässrigem Methanol, zweckmässig bei Raumtemperatur umgesetzt werden.

Die in dem obigen Verfahren eingesetzten Ausgangsmaterialien sind bekannte Verbindungen oder Analoge bekannter Verbindungen, die in ähnlicher Weise wie die bekannten Verbindungen, z.B. wie nachfolgend in den Beispielen beschrieben, hergestellt werden können.

Die Verbindungen der Formel I und deren Tautomere besitzen antivirale Aktivität und können zur Kontrolle oder Verhütung viraler Infektionen, z.B. von viralen Herpes simplex-Infektionen verwendet werden.

Die in vitro Aktivität der Verbindungen der Formel I und deren Tautomere bei der Hemmung von Herpes simplex-Virus Typ 2 (HSV-2)-Thymidinkinase kann mittels des folgenden Tests gezeigt werden:

In diesem Test enthält ein Versuchsgemisch 50 mM Tris-HCl, pH 8, 5 mM Magnesiumchlorid, 5 mM ATP, 0,3 µM $^3$H-Thymidin (50 Ci/mMol), in geeigneter Weise verdünnten Thymidinkinaseextrakt und verschiedene Konzentrationen von Verbindungen der Formel I oder Tautomeren davon in einem Gesamtvolumen von 100 µl. Die Testlösungen werden 30 Minuten bei 37°C inkubiert und die Reaktion durch 2 Minuten langes Eintauchen in kochendes Wasser beendet. Von jeder Testlösung werden dann 85 µl auf Cellulosepapier-Discs getrocknet und das unphosphorylierte $^3$H-Thymidin durch Waschen in 4 mM Ammoniumformat entfernt. Die an die Discs gebunden gebliebene Radioaktivität wird dann durch Szintillationsspektrophotometrie gemessen. Der Grad der Hemmung bei jeder Konzentration der Verbindung der Formel I wird als Prozentsatz der Kontrollreaktion (100%) nach Abzug eines gemessenen Blindwertes ausgedrückt, der die Menge Radioaktivität darstellt, die an die Discs aus einem Versuch mit Hitze-inaktivierten Enzymen gebunden wird. Der $IC_{50}$-Wert, nämlich die Konzentration der Verbindung der Formel I oder dessen Tautomeren, die die Enzymaktivität zu 50% hemmt, wird dann berechnet. Die mit repräsentativen Verbindungen der Formel I erhaltenen Resultate sind in der nachfolgenden Tabelle zusammengestellt:

Tabelle

| Verbindung von Beispiel No. | $IC_{50}$ (µM) |
|---|---|
| 3b | 0.09 |
| 4b | 0.06 |
| 4c | 0.08 |

Die Verbindungen der Formel I und deren Tautomere können in Form pharmazeutischer Präparate, die diese Verbindungen zusammen mit einem verträglichen pharmazeutischen Träger enthalten, als Medikamente verwendet werden. Die Träger können organische oder anorganische Materialien sein, die für enterale, z.B. orale, oder parenterale Verabreichung geeignet sind. Beispiele solcher Träger sind Wasser, Gelatine, Gummi arabicum, Lactose, Stärke, Magnesiumstearat, Talkum, vegetabile Oele, Polyalkylenglykole und Vaseline. Die pharmazeutischen Präparate können in fester Form, z.B. als Tabletten, Dragées, Suppositorien oder Kapseln, oder in flüssiger Form, z.B. als Lösungen, Suspensionen oder Emulsionen vorliegen; sie können üblichen pharmazeutischen Operationen, z.B. Sterilisierung unterworfen werden und/oder können Hilfsstoffe, z.B. Konservierungs-, Stabilisierungs-, Befeuchtungsmittel oder Emulgatoren, Salze zur Veränderung des osmotischen Druckes oder Puffer enthalten. Sie können auch andere therapeutisch wertvolle Substanzen enthalten.

Die Verbindungen der Formel I und deren Tautomere können an Erwachsene in einer täglichen Dosierung von etwa 1-1000 mg, vorzugsweise etwa 5-500 mg verabreicht werden. Die tägliche Dosis kann in einer Einzeldosis oder in Teildosen verabreicht werden. Der obige Dosierungsbereich ist nur beispielhaft zu verstehen und kann nach oben und unten in Abhängigkeit von Faktoren wie der einzelnen zu verabreichenden Verbindung, der Verabreichungsart, der Schwere der zu behandelnden Erkrankung und dem Zustand des Patienten variiert werden.

Beispiel 1

Eine Lösung von 396 mg 5'-O-(p-Toluolsulfonyl)thymidin und 1,5 ml 4-Chlorbenzylamin in 2 ml DMF wurde 2 Stunden bei 80 °C gerührt. Das Lösungsmittel wurde abgedampft und der Rückstand mit Diäthyläther verrieben. Der erhaltene Feststoff wurde aus Aethanol umkristallisiert und lieferte 56 mg 5'-(4-Chlorbenzylamino)-5'-deoxythymidin, Schmelzpunkt 179-180 °C.

Beispiel 2

2,5 2'-Deoxy-5-äthyl-5'-O-(p-toluolsulfonyl)uridin wurden in 15 ml DMF gelöst und 2-Brombenzylamin gegeben. Das Gemisch wurde 5 Stunden unter Stickstoff auf 80 °C erwärmt. Das Lösungsmittel wurde abgedampft und der Rückstand erneut mit Toluol abgedampft, wobei ein Oel erhalten wurde. Dieses wurde in 20 ml Wasser/Methanol (1:3) aufgenommen und 1,5 Stunden im Kühlschrank belassen. Der auskristallisierende Feststoff wurde abfiltriert und mit Wasser/Methanol (1:3) gewa schen und lieferte 0,99 g 5'-(2-Brombenzylamino)-2',5'-dideoxy-5-äthyluridin, Schmelzpunkt 144-146 °C.

Das Ausgangsmaterial, 2'-Deoxy-5-äthyl-5'-O-(p-toluolsulfonyl)uridin wurde wie folgt hergestellt:

26 g 2'-Deoxy-5-äthyluridin wurden in 400 ml Pyridin gelöst. Die Lösung wurde auf 0 °C gekühlt und unter Rühren nach und nach mit 20 g p-Toluolsulfonylchlorid versetzt. Es wurde noch 1 Stunde bei 0 °C gerührt und das Gemisch dann über Nacht bei 4 °C belassen. Das Lösungsmittel wurde abgedampft und der Rückstand mit Toluol abgedampft. Der Rückstand wurde mit 200 ml Methanol geschüttelt und 2,5 Stunden im Kühlschrank stehen gelassen, wobei ein Feststoff erhalten wurde, der abfiltriert, mit Methanol gewaschen und getrocknet wurde. Man erhielt 18 g eines Produkts, das nach Umkristallisation aus 450 ml Aethanol 13 g 2'-Deoxy-5-äthyl-5'-O-(p-toluolsulfonyl)uridin, Schmelzpunkt 189-190 °C (Zersetzung) lieferte.

Beispiel 3

In Analogie zu Beispiel 1 wurden erhalten:

a) aus 2′-Deoxy-5-äthyl-5′-O-(p-toluolsulfonyl)uridin und 2-Bromphenyläthylamin:
5′-[2-(2-Bromophenyl)äthylamino]-2',5′-dideoxy-5-äthyluridin, Schmelzpunkt 109-111 ° C;

b) aus 2′-Deoxy-5-äthyl-5′-O-(p-toluolsulfonyl)uridin und 2,6-Dichlorphenyläthylamin:
5′-[2-(2,6-Dichlorphenyl)äthylamino]-2′,5′-dideoxy-5-äthyluridin, Schmelzpunkt 127-128 ° C; und

c) aus 2′-Deoxy-5-äthyl-5′-O-(p-toluolsulfonyl)uridin und 2,6-Dimethylphenyläthylamin:
2′,5′-Dideoxy-5-äthyl-5′-[2-(2,6-dimethylphenyl)äthylamino]uridin, Schmelzpunkt 73-76 ° C.

Beispiel 4

In Analogie zu Beispiel 2 wurden erhalten:

a) aus 2′-Deoxy-5-äthyl-5′-O-(p-toluolsulfonyl)uridin und p-Chlorbenzylamin:
5′-(4-Chlorbenzylamino)-2′,5′-dideoxy-5-äthyluridin, Schmelzpunkt 154-156 ° C;

b) aus 2′-Deoxy-5-äthyl-5′-O-(p-toluolsulfonyl)uridin und 2,3-Dichlorbenzylamin:
5′-(2,3-Dichlorbenzylamino)-2′,5'-dideoxy-5-äthyluridin, Schmelzpunkt 158-160 ° C;

c) aus 2′-Deoxy-5-äthyl-5′-O-(p-toluolsulfonyl)uridin und 2,6-Dichlorbenzylamin:
5′-(2,6-Dichlorbenzylamino)-2′,5'-dideoxy-5-äthyluridin, Schmelzpunkt 208-210 ° C;

d) aus 2′-Deoxy-5-äthyl-5′-O-(p-toluolsulfonyl)uridin und 1(R)-Phenyläthylamin:
2′,5′-Dideoxy-5-äthyl-5′-[1(R)-phenyläthylamino]uridin, Schmelzpunkt 153 ° C, $[\alpha]_D^{20}$ = +27,1 ° (c = 0,25% in DMSO);

e) aus 2′-Deoxy-5-äthyl-5′-O-(p-toluolsulfonyl)uridin und 1(S)-Phenyläthylamin:
2′,5′-Dideoxy-5-äthyl-5′-[1(S)-phenyläthylamino]uridin, Schmelzpunkt 136 ° C, $[\alpha]_D^{20}$ = -19,5 ° (c = 0,25% in DMSO);

f) aus 2′-Deoxy-5-äthyl-5′-O-(p-toluolsulfonyl)uridin und N-Benzylmethylamin:
2′,5′-Dideoxy-5-äthyl-5′-(N-methylbenzylamino)uridin, Schmelzpunkt 135-137 ° C;

g) aus 2′-Deoxy-5-äthyl-5′-O-(p-toluolsulfonyl)uridin und Phenyläthylamin:
2′,5′-Dideoxy-5-äthyl-5′-(phenyläthylamino)uridin, Schmelzpunkt 123-125 ° C;

h) aus 2′-Deoxy-5-äthyl-5′-O-(p-toluolsulfonyl)uridin und 2-(2-Methylphenyl)äthylamin:
2′,5′-Dideoxy-5-äthyl-5-[2-(2-methylphenyl)äthylamino]uridin, Schmelzpunkt 117 ° C;

i) aus 2′-Deoxy-5-äthyl-5′-O-(p-toluolsulfonyl)uridin und N-Methyl-2-phenyläthylamin:
2′,5′-Dideoxy-5-äthyl-5′-N-methylphenyläthylamino)uridin, Schmelzpunkt 110 ° C; und

j) aus 2′-Deoxy-5-äthyl-5′-O-(p-toluolsulfonyl)uridin und d-Amphetamin:
5′-[1(S)-Benzyläthylamino)-2′5′-dideoxy-5-äthyluridin, Schmelzpunkt 150 ° C; $[\alpha]_D^{20}$ = +23,4 ° (c = 0,25% in DMSO).

Beispiel 5

Eine Lösung von 0,51 g 5′-Amino-2′,5′-dideoxy-5-äthyluridin in 15 ml Aethanol wurde unter Stickstoff mit 0,21 ml Benzaldehyd umgesetzt. Danach wurde eine Aufschlämmung von 75 mg 5% Pd/C-Katalysator in 5 ml Aethanol zugesetzt und das Gemisch bei Raumtemperatur und Atmosphärendruck 4,5 Stunden hydriert. Der Katalysator wurde abfiltriert, das Filtrat eingedampft und der erhaltene Schaum mit Diäthyläther verrieben, wobei 0,57 g eines Feststoffs erhalten wurden. Der Feststoff wurde in Methylenchlorid/Methanol (9:1) aufgenommen und an Silicagel chromatographiert unter Elution mit Methylenchlorid/Methanol. Die das Produkt enthaltenden Fraktionen wurden vereinigt und eingedampft. Der Rückstand wurde mit Diäthyläther verrieben und abfiltriert und lieferte 0,45 g 5′-Benzylamino-2′,5′-dideoxy-5-äthyluridin, Schmelzpunkt 121-124 ° C.

Beispiel 6

In Analogie zu Beispiel 5 wurden erhalten:

a) aus 5′-Amino-2′,5′-dideoxy-5-äthyluridin und o-Tolualdehyd:
2′,5′-Dideoxy-5-äthyl-5′-(2-methylbenz lamino)uridin, Schmelzpunkt 157-158 ° C und

b) aus 5′-Amino-5′-deoxythymidin und Benzaldehyd:
5′-Benzylamino-5′-deoxythymidin, Schmelzpunkt 133-135 ° C.

Beispiel 7

Zu einer eisgekühlten Lösung von 0,45 g 5′-Amino-2′,5′-dideoxy-5-äthyluridin in 28 ml Methanol und 9 ml Wasser wurden 0,17 g Natriumcyanborhydrid und anschliessend 0,5 g 2-(2,4-Dichlorphenoxy)-propionaldehyd gegeben. Das Gemisch wurde auf Raumtemperatur aufwärmen gelassen und dann 3 Tage bei Raumtemperatur gerührt. Die Lösung wurde zur Entfernung des Methanols konzentriert, mit 20 ml Wasser verdünnt und dreimal mit 15 ml Methylenchlorid extrahiert. Die vereinigten Extrakte wurden über Natriumsulfat getrocknet und eingedampft. Der Rückstand wurde an Silicagel mit Methanol/Methylenchlorid (1:12) chromatographiert. Man erhielt 0,26 g 2′,5′-Dideoxy-5-äthyl-5′ -[2(RS)-(2,4-dichlorphenoxy)-propylamino]uridin, Schmelzpunkt 51-61 ° C.

Der als Ausgangsmaterial verwendete 2-(2,4-Dichlorphenoxy)propionaldehyd wurde wie folgt hergestellt:

43 ml einer 1M Lösung von Boran in Tetrahydrofuran (THF) wurden unter Stickstoff und Eiskühlung gerührt, während eine Lösung von 6,8 g 2-(2,4-Dichlorphenoxy)propionsäure in 40 ml THF tropfenweise zugesetzt wurde. Das Gemisch wurde 1 Stunde zum Rückfluss erhitzt und dann auf Raumtemperatur gekühlt. 22 ml einer gesättigten Lösung von Chlorwasserstoff in Methanol wurden tropfenweise zugesetzt und die Lösung 1 Stunde zum Rückfluss erhitzt. Das Gemisch wurde eingedampft und der Rückstand mit 22 ml einer gesättigten Lösung von Chlorwasserstoff in Methanol versetzt, die Lösung 1 Stunde zum Rückfluss erhitzt, das Gemisch eingedampft und der Rückstand zwischen 100 ml gesättigter Natriumbicarbonatlösung und 100 ml Methylenchlorid verteilt. Die wässrige Phase wurde mit 100 ml Methylenchlorid extrahiert. Die vereinigten organischen Extrakte wurden über Natriumsulfat getrocknet und eingedampft und lieferten 6,4 g 2-(2,4-Dichlorphenoxy)propanol als gelbes Oel.

4,68 g DMSO wurden unter Rühren und unter Stickstoff zu einer Lösung von 3,81 g Oxalylchlorid in 75 ml Methylenchlorid bei -60° bis -50° C gegeben. Nach 2 Minuten wurde eine Lösung von 6,4 g 2-(2,4-Dichlorphenoxy)propanol in 40 ml Methylenchlorid zugesetzt. Das Gemisch wurde weitere 15 Minuten bei -60° C gerührt und dann mit 13,7 g Triäthylamin versetzt. Nach 5 Minuten bei -60° C wurde das Gemisch auf Raumtemperatur aufwärmen gelassen und mit 150 ml Wasser versetzt. Die Phasen wurden getrennt, die wässrige Phase mit 150 ml Methylenchlorid extrahiert und die vereinigten organischen Extrakte nacheinander mit Natriumchloridlösung, verdünnter Salzsäure, Wasser, verdünnter Natriumcarbonatlösung und Wasser gewaschen, über Natriumsulfat getrocknet und eingedampft. Der Rückstand wurde durch Chromatographie an Silicagel mit Aethylacetat/n-Hexan (1:4) gereinigt. Man erhielt 1,4 g 2-(2,4-Dichlorphenoxy)-propionaldehyd, Schmelzpunkt 87-88° C.

Das folgende Beispiel illustriert ein pharmazeutisches Präparat, das eine Verbindung der Formel I enthält:

Tabletten mit den folgenden Inhaltsstoffen können in üblicher Weise hergestellt werden:

| Inhaltsstoff | | pro Tablette |
|---|---|---|
| Verbindung der Formel I | | 100 mg |
| Lactose | | 70 mg |
| Maisstärke | | 70 mg |
| Polyvinylpyrrolidon | | 5 mg |
| Magnesiumstearat | | 5 mg |
| | Tablettengewicht | 250 mg |

**Patentansprüche**

**Patentansprüche für folgende Vertragsstaaten : BE, CH, DE, FR, GB, IT, LI, LU, NL, SE**

**1.** Verbindungen der allgemeinen Formel

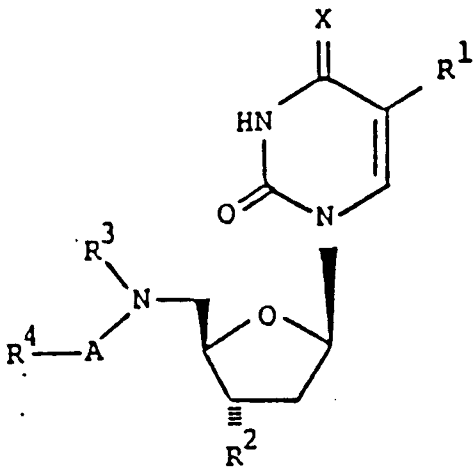

I

worin A $C_{1-8}$-Alkylen, $R^1$ Halogen, $C_{1-4}$-Alkyl oder Halo-($C_{1-4}$-alkyl), $R^2$ Wasserstoff, Hydroxy oder Acyloxy, $R^3$ Wasserstoff oder $C_{1-4}$-Alkyl, $R^4$ Aryl oder Aryloxy und X Sauerstoff oder NH bedeutet, wobei die Acyloxygruppe sich von einer aliphatischen, cycloaliphatischen, araliphatischen oder aromatischen Carbonsäure ableitet, und Aryl gegebenenfalls einen oder mehrere Substituenten aus der Reihe von Halogen, OH, $C_{1-4}$-Alkyl, $C_{1-4}$-Alkoxy, $CF_3$, $NO_2$ und $C_6H_5$ enthaltendes Phenyl ist, und Tautomere davon.

**2.** Verbindungen gemäss Anspruch 1, wobei $R^4$ Aryl ist.

**3.** Verbindungen gemäss Anspruch 1 oder 2, wobei A $C_{1-4}$-Alkylen ist.

**4.** Verbindungen gemäss den Ansprüchen 1, 2 oder 3, wobei $R^1$ $C_{1-4}$-Alkyl ist.

**5.** Verbindungen gemäss einem der Ansprüche 1-4, wobei $R^2$ Hydroxy ist.

**6.** Verbindungen gemäss einem der Ansprüche 1-5, wobei $R^3$ Wasserstoff oder Methyl ist.

**7.** Verbindungen gemäss einem der Ansprüche 1-6, wobei $R^4$ Dihalophenyl ist.

**8.** Verbindungen gemäss einem der Ansprüche 1-7, wobei X Sauerstoff ist.

**9.** Verbindungen gemäss einem der Ansprüche 1-8, wobei A $-CH_2-$, $-CH_2CH_2-$, $-CH(CH_3)-$ oder $-CH_2-CH(CH_3)-$, $R^1$ Aethyl, $R^2$ Hydroxy, $R^3$ Wasserstoff oder Methyl, $R^4$ 2,3- oder 2,6-Dichlorphenyl und X Sauerstoff sind.

**10.** Die Verbindungen
5′-(2,6-Dichlorbenzylamino)-2′,5′-dideoxy-5-äthyluridin,
5′-[2-(2,6-Dichlorphenyl)äthylamino]-2′,5′-dideoxy-5-eähyluridin und
5′-(2,3-Dichlorbenzylamino)-2′,5′-dideoxy-5-äthyluridin.

**11.** Die Verbindungen
5′-(4-Chlorbenzylamino)-5′-deoxythymidin,
5′-(2-Brombenzylamino)-2′,5′-dideoxy-5-äthyluridin,
5′-[2-(2-Bromophenyl)äthylamino]-2′,5′-dideoxy-5-äthyluridin,
2′,5′-Dideoxy-5-äthyl-5′-[2-(2,6-dimethylphenyl)äthylamino]uridin,
5′-(4-Chlorbenzylamino)-2',5′-dideoxy-5-äthyluridin,
2′,5′-Dideoxy-5-äthyl-5′-[1(R)-phenyläthylamino]uridin,
2′,5′-Dideoxy-5-äthyl-5′-[1(S)-phenyläthylamino]uridin,
2′,5′-Dideoxy-5-äthyl-5′-(N-methylbenzylamino)uridin,
2',5′-Dideoxy-5-äthyl-5′-(phenyläthylamino)uridin,
2′,5′-Dideoxy-5-äthyl-5′-[2-(2-methylphenyl)äthylamino]uridin,
2′,5′-Dideoxy-5-äthyl-5′-(N-methylphenyläthylamino)uridin,

5'-[1(S)-Benzyläthylamino]-2',5'-dideoxy-5-äthyluridin,
5'-Benzylamino-2',5'-dideoxy-5-äthyluridin,
2',5'-Dideoxy-5-äthyl-5'-(2-methylbenzylamino)uridin und
5'-Benzylamino-5'-deoxythymidin.

**12.** 2',5'-Dideoxy-5-äthyl-5' -[2(RS)-(2,4-dichlorphenoxy)propylamino]uridin.

**13.** Die Verbindungen gemäss den Ansprüchen 1-12 zur Verwendung als Heilmittel, insbesondere als Mittel gegen Viren.

**14.** Verfahren zur Herstellung der Verbindungen der allgemeinen Formel I und deren Tautomeren, dadurch gekennzeichnet, dass man

a) eine Verbindung der Formel

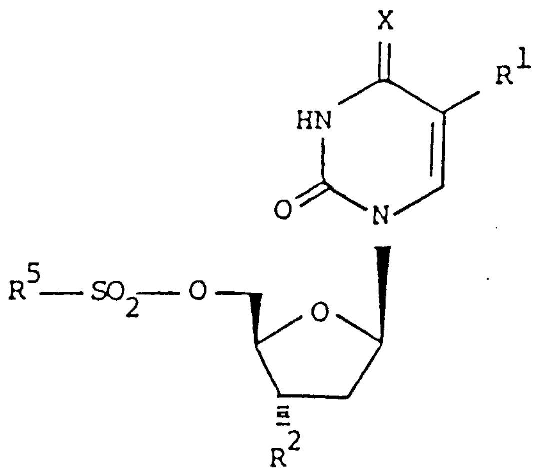

II

worin $R^1$, $R^2$ und X die obige Bedeutung haben und $R^5$ $C_{1-4}$-Alkyl oder Aryl ist, oder ein Tautomer davon mit einer Verbindung der Formel

$R^3$-NH-A-$R^4$ III

worin A, $R^3$ und $R^4$ die obige Bedeutung haben, bei erhöhter Temperatur umsetzt oder

b) zur Herstellung einer Verbindung der Formel I, in der X O ist oder eines Tautomeren davon, die Gruppe $R^4$-A- in eine Verbindung der Formel

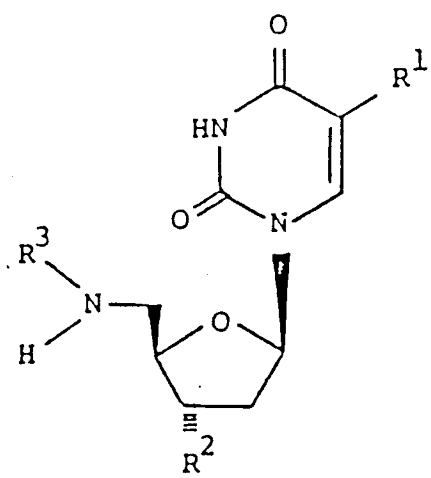

IV

worin $R^1$, $R^2$ und $R^3$ die obige Bedeutung haben, oder ein Tautomer davon durch reduktive Alkylierung einführt.

**15.** Pharmazeutische, insbesondere anti-viral wirksame Präparate, enthaltend eine Verbindung gemäss den Ansprüchen 1-12 und ein pharmazeutisches Trägermaterial.

**16.** Verwendung einer Verbindung gemäss den Ansprüchen 1-12 zur Herstellung von Arzneimitteln zur Kontrolle oder Prophylaxe viraler Infektionen.

**Patentansprüche für folgende Vertragsstaaten : AT, ES, GR**

**1.** Verfahren zur Herstellung von Verbindungen der allgemeinen Formel

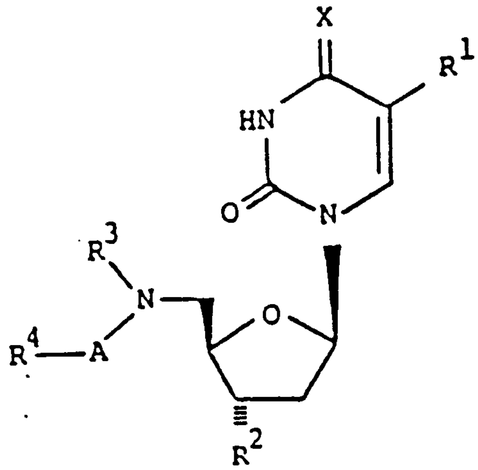

I

worin A $C_{1-8}$-Alkylen, $R^1$ Halogen, $C_{1-4}$-Alkyl oder Halo-($C_{1-4}$-alkyl), $R^2$ Wasserstoff, Hydroxy oder Acyloxy, $R^3$ Wasserstoff oder $C_{1-4}$-Alkyl, $R^4$ Aryl oder Aryloxy und X Sauerstoff oder NH bedeutet, wobei die Acylgruppe sich von einer aliphatischen, cycloaliphatischen, araliphatischen oder aromatischen Carbonsäure ableitet, und Aryl gegebenenfalls einen oder mehrere Substituenten aus der Reihe von Halogen, OH, $C_{1-4}$-Alkyl, $C_{1-4}$-Alkoxy, $CF_3$, $NO_2$ und $C_6H_5$ enthaltendes Phenyl ist,
und Tautomere davon, dadurch gekennzeichnet, dass man
a) eine Verbindung der Formel

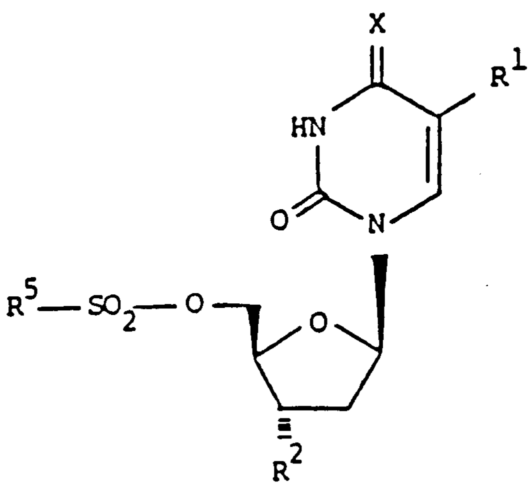

II

worin $R^1$, $R^2$ und X die obige Bedeutung haben und $R^5$ $C_{1-4}$-Alkyl oder Aryl ist,
oder ein Tautomer davon mit einer Verbindung der Formel

$R^3$-NH-A-$R^4$ III

worin A, $R^3$ und $R^4$ die obige Bedeutung haben,
bei erhöhter Temperatur umsetzt oder
b) zur Herstellung einer Verbindung der Formel I, in der X O ist oder eines Tautomeren davon, die

Gruppe $R^4$-A in eine Verbindung der Formel

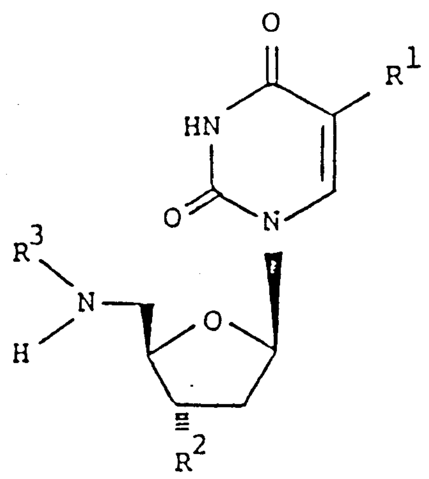

IV

worin $R^1$, $R^2$ und $R^3$ die obige Bedeutung haben, oder ein Tautomer davon durch reduktive Alkylierung einführt.

**2.** Verfahren gemäss Anspruch 1, wobei $R^4$ Aryl ist.

**3.** Verfahren gemäss Anspruch 1 oder 2, wobie A C $_{1\text{-}4}$-Alkylen ist.

**4.** Verfahren gemäss den Ansprüchen 1, 2 oder 3, wobei $R^1$ $C_{1\text{-}4}$-Alkyl ist.

**5.** Verfahren gemäss einem der Ansprüche 1-4, wobei $R^2$ Hydroxy ist.

**6.** Verfahren gemäss einem der Ansprüche 1-5, wobei $R^3$ Wasserstoff oder Methyl ist.

**7.** Verfahren gemäss einem der Ansprüche 1-6, wobei $R^4$ Dihalophenyl ist.

**8.** Verfahren gemäss einem der Ansprüche 1-7, wobei X Sauerstoff ist.

**9.** Verfahren gemäss einem der Ansprüche 1-8, wobei A $-CH_2-$, $-CH_2CH_2-$, $-CH(CH_3)-$ oder $-CH_2-CH(CH_3)-$, $R^1$ Aethyl, $R^2$ Hydroxy, $R^3$ Wasserstoff oder Methyl, $R^4$ 2,3- oder 2,6-Dichlorphenyl und X Sauerstoff sind.

**10.** Verfahren gemäss Anspruch 1 zur Herstellung der Verbindungen
5′-(2,6-Dichlorbenzylamino)-2′,5′-dideoxy-5-äthyluridin,
5′-[2-(2,6-Dichlorphenyl)äthylamino]-2′,5′-dideoxy-5-eähyluridin und
5′-(2,3-Dichlorbenzylamino)-2′,5′-dideoxy-5-äthyluridin.

**11.** Verfahren gemäss Anspruch 2 zur Herstellung der Verbindungen
5′-(4-Chlorbenzylamino)-5′-deoxythymidin,
5′-(2-Brombenzylamino)-2′,5′-dideoxy-5-äthyluridin,
5′-[2-(2-Bromophenyl)äthylamino]-2′,5′-dideoxy-5-äthyluridin,
2′,5′-Dideoxy-5-äthyl-5′-[2-(2,6-dimethylphenyl)äthylamino]uridin,
5′-(4-Chlorbenzylamino)-2′,5'-dideoxy-5-äthyluridin,
2′,5′-Dideoxy-5-äthyl-5′-[1(R)-phenyläthylamino]uridin,
2′,5′-Dideoxy-5-äthyl-5′-[1(S)-phenyläthylamino]uridin,
2′,5′-Dideoxy-5-äthyl-5′-(N-methylbenzylamino)uridin,
2′,5′-Dideoxy-5-äthyl-5′-(phenyläthylamino)uridin,
2′,5′-Dideoxy-5-äthyl-5′-[2-(2-methylphenyl)äthylamino]-uridin,
2′,5′-Dideoxy-5-äthyl-5′-(N-methylphenyläthylamino)uridin,
5′-[1(S)-Benzyläthylamino]-2′,5'-dideoxy-5-äthyluridin,
5′-Benzylamino-2′,5′-dideoxy-5-äthyluridin,

2',5'-Dideoxy-5-äthyl-5'-(2-methylbenzylamino)uridin und
5'-Benzylamino-5'-deoxythymidin.

**12.** Verfahren gemäss Anspruch 1 zur Herstellung der Verbindung 2',5'-Dideoxy-5-äthyl-5' -[2(RS)-(2,4-dichlor phenoxy)propylamino]uridin.

**13.** Verwendung einer Verbindung gemäss den Ansprüchen 1-12 zur Herstellung von Arzneimitteln zur Kontrolle oder Prophylaxe viraler Infektionen.

**14.** Verfahren zur Herstellung von pharmazeutischen Präparaten, dadurch gekennzeichnet, dass man eine Verbindung der Formel I in eine galenische Verabreichungsform bringt.

**Claims**

**Claims for the following Contracting States : BE, CH, DE, FR, GB, IT, LI, LU, NL, SE**

**1.** Compounds of the general formula

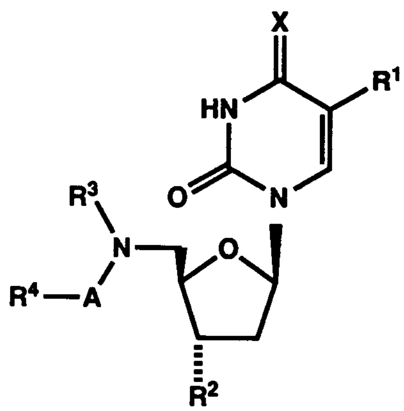

I

wherein A signifies $C_{1-8}$-alkylene, $R^1$ signifies halogen, $C_{1-4}$-alkyl or halo-($C_{1-4}$-alkyl), $R^2$ signifies hydrogen, hydroxy or acyloxy, $R^3$ signifies hydrogen or $C_{1-4}$-alkyl, $R^4$ signifies aryl or aryloxy and X signifies oxygen or NH, whereby the acyloxy group is derived from an aliphatic, cycloaliphatic, araliphatic or aromatic carboxylic acid and aryl is phenyl optionally containing one or more substituents from the series of halogen, OH, $C_{1-4}$-alkyl, $C_{1-4}$-alkoxy, $CF_3$, $NO_2$ and $C_6H_5$,
and tautomers thereof.

**2.** Compounds according to claim 1, wherein $R^4$ is aryl.

**3.** Compounds according to claim 1 or 2, wherein A is $C_{1-4}$-alkylene.

**4.** Compounds according to claim 1, 2 or 3, wherein $R^1$ is $C_{1-4}$-alkyl.

**5.** Compounds according to any one of claims 1-4, wherein $R^2$ is hydroxy.

**6.** Compounds according to any one of claims 1-5, wherein $R^3$ is hydrogen or methyl.

**7.** Compounds according to any one of claims 1-6, wherein $R^4$ is dihalophenyl.

**8.** Compounds according to any one of claims 1-7, wherein X is oxygen.

**9.** Compounds according to any one of claims 1-8, wherein A is $-CH_2-$, $-CH_2CH_2-$, $-CH(CH_3)-$ or $-CH_2-CH(CH_3)-$, $R^1$ is ethyl, $R^2$ is hydroxy, $R^3$ is hydrogen or methyl, $R^4$ is 2,3- or 2,6-dichlorophenyl and X is oxygen.

**10.** The compounds

5'-(2,6-dichlorobenzylamino)-2',5'-dideoxy-5-ethyluridine,
5'-[2-(2,6-dichlorophenyl)ethylamino]-2',5'-dideoxy-5-ethyluridine and
5'-(2,3-dichlorobenzylamino)-2',5'-dideoxy-5-ethyluridine.

**11.** The compounds
5'-(4-chlorobenzylamino)-5'-deoxythymidine,
5'-(2-bromobenzylamino)-2',5'-dideoxy-5-ethyluridine,
5'-[2-(2-bromophenyl)ethylamino]-2',5'-dideoxy-5-ethyluridine,
2',5'-dideoxy-5-ethyl-5'-[2-(2,6-dimethylphenyl)ethylamino]uridine,
5'-(4-chlorobenzylamino)-2',5'-dideoxy-5-ethyluridine,
2',5'-dideoxy-5-ethyl-5'-[1(R)-phenylethylamino]uridine,
2',5'-dideoxy-5-ethyl-5'-[1(S)-phenylethylamino]uridine,
2',5'-dideoxy-5-ethyl-5'-(N-methylbenzylamino)uridine,
2',5'-dideoxy-5-ethyl-5'-(phenylethylamino)uridine,
2',5'-dideoxy-5-ethyl-5'-[2-(2-methylphenyl)ethylamino]uridine,
2',5'-dideoxy-5-ethyl-5'-(N-methylphenylethylamino)uridine,
5'-[1(S)-benzylethylamino]-2',5'-dideoxy-5-ethyluridine,
5'-benzylamino-2',5'-dideoxy-5-ethyluridine,
2',5'-dideoxy-5-ethyl-5'-(2-methylbenzylamino)uridine and 5'-benzylamino-5'-deoxythymidine.

**12.** 2,5'-Dideoxy-5-ethyl-5'-[2(RS)-(2,4-dichlorophenoxy)propylamino]uridine.

**13.** The compounds according to claims 1-12 for use as medicaments, especially as antiviral medicaments.

**14.** A process for the manufacture of the compounds of general formula I and their tautomers, characterized by
a) reacting a compound of the formula

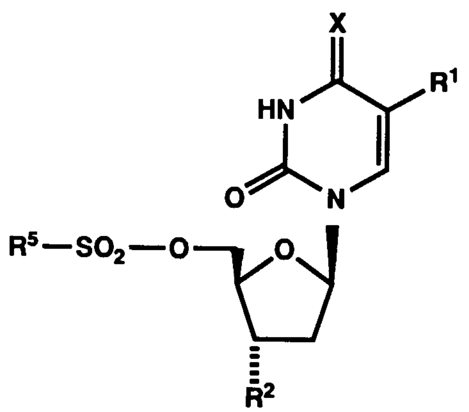

II

wherein $R^1$, $R^2$ and X have the above significance and $R^5$ is $C_{1-4}$-alkyl or aryl, or a tautomer thereof with a compound of the formula

$R^3$-NH-A-$R^4$ III

wherein A, $R^3$ and $R^4$ have the above significance, at an elevated temperature, or
b) for the manufacture of a compound of formula I in which X is O or a tautomer thereof, introducing the group $R^4$-A into a compound of the formula

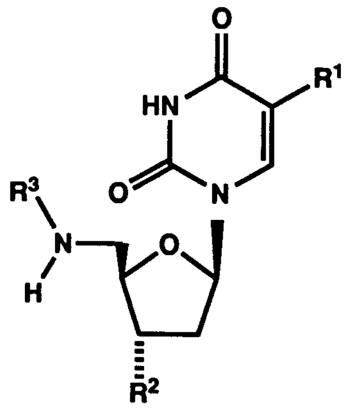

IV

wherein $R^1$, $R^2$ and $R^3$ have the above significance, or a tautomer thereof by reductive alkylation.

**15.** A pharmaceutical preparation, especially an antiviral preparation, containing a compound according to claims 1-12 and a pharmaceutical carrier material.

**16.** The use of a compound according to claims 1-12 for the manufacture of medicaments for the control or prophylaxis or viral infections.

**Claims for the following Contracting States : AT, ES, GR**

**1.** A process for the manufacture of compounds of the general formula

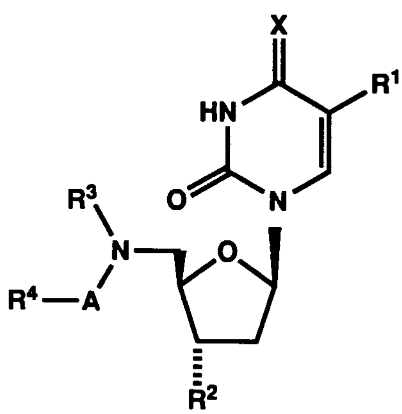

I

wherein A signifies $C_{1-8}$-alkylene, $R^1$ signifies halogen, $C_{1-4}$-alkyl or halo-($C_{1-4}$-alkyl), $R^2$ signifies hydrogen, hydroxy or acyloxy, $R^3$ signifies hydrogen or $C_{1-4}$-alkyl, $R^4$ signifies aryl or aryloxy and X signifies oxygen or NH, whereby the acyloxy group is derived from an aliphatic, cycloaliphatic, araliphatic or aromatic carboxylic acid and aryl is phenyl optionally containing one or more substituents from the series of halogen, OH, $C_{1-4}$-alkyl, $C_{1-4}$-alkoxy, $CF_3$, $NO_2$ and $C_6H_5$,
and tautomers thereof, characterized by

a) reacting a compound of the formula

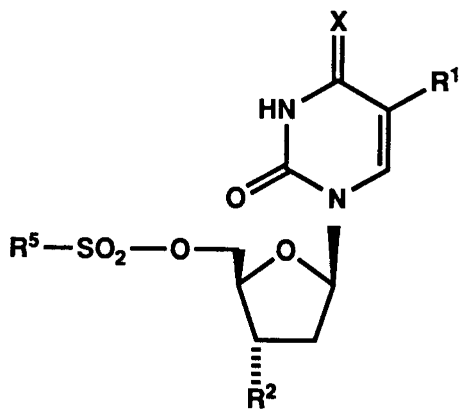

II

wherein $R^1$, $R^2$ and X have the above significance and $R^5$ is $C_{1-4}$-alkyl or aryl, or a tautomer thereof with a compound of the formula

$R^3$-NH-A-$R^4$ III

wherein A, $R^3$ and $R^4$ have the above significance, at an elevated temperature, or
b) for the manufacture of a compound of formula I in which X is O or a tautomer thereof, introducing the group $R^4$-A into a compound of the formula

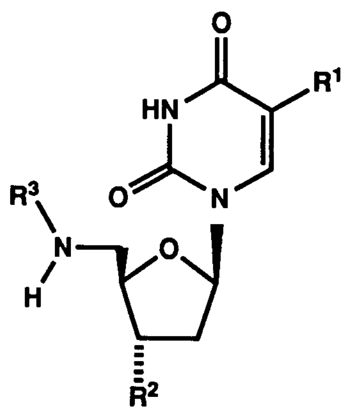

IV

wherein $R^1$, $R^2$ and $R^3$ have the above significance, or a tautomer thereof by reductive alkylation.

**2.** A process according to claim 1, wherein $R^4$ is aryl.

**3.** A process according to claim 1 or 2, wherein A is $C_{1-4}$-alkylene.

**4.** A process according to claim 1, 2 or 3, wherein $R^1$ is $C_{1-4}$-alkyl.

**5.** A process according to any one of claims 1-4, wherein $R^2$ is hydroxy.

**6.** A process according to any one of claims 1-5, wherein $R^3$ is hydrogen or methyl.

**7.** A process according to any one of claims 1-6, wherein $R^4$ is dihalophenyl.

**8.** A process according to any one of claims 1-7, wherein X is oxygen.

**9.** A process according to any one of claims 1-8, wherein A is $-CH_2-$, $-CH_2CH_2-$, $-CH(CH_3)-$ or $-CH_2-CH-$

$(CH_3)$-, $R^1$ is ethyl, $R^2$ is hydroxy, $R^3$ is hydrogen or methyl, $R^4$ is 2,3- or 2,6-dichlorophenyl and X is oxygen.

**10.** A process according to claim 1 for the manufacture of the compounds
5'-(2,6-dichlorobenzylamino)-2',5'-dideoxy-5-ethyluridine,
5'-[2-(2,6-dichlorophenyl)ethylamino]-2',5'-dideoxy-5-ethyluridine and
5'-(2,3-dichlorobenzylamino)-2',5'-dideoxy-5-ethyluridine.

**11.** A process according to claim 2 for the manufacture of the compounds
5'-(4-chlorobenzylamino)-5'-deoxythymidine,
5'-(2-bromobenzylamino)-2',5'-dideoxy-5-ethyluridine,
5'-[2-(2-bromophenyl)ethylamino]-2',5'-dideoxy-5-ethyluridine,
2',5'-dideoxy-5-ethyl-5'-[2-(2,6-dimethylphenyl)ethylamino]uridine,
5'-(4-chlorobenzylamino)-2',5'-dideoxy-5-ethyluridine,
2',5'-dideoxy-5-ethyl-5'-[1(R)-phenylethylamino]uridine,
2',5'-dideoxy-5-ethyl-5'-[1(S)-phenylethylamino]uridine,
2',5'-dideoxy-5-ethyl-5'-(N-methylbenzylamino)uridine,
2',5'-dideoxy-5-ethyl-5'-(phenylethylamino)uridine,
2',5'-dideoxy-5-ethyl-5'-[2-(2-methylphenyl)ethylamino]uridine,
2',5'-dideoxy-5-ethyl-5'-(N-methylphenylethylamino)uridine,
5'-[1(S)-benzylethylamino]-2',5'-dideoxy-5-ethyluridine,
5'-benzylamino-2',5'-dideoxy-5-ethyluridine,
2',5'-dideoxy-5-ethyl-5'-(2-methylbenzylamino)uridine and 5'-benzylamino-5'-deoxythymidine.

**12.** A process according to claim 1 for the manufacture of the compound 2',5'-dideoxy-5-ethyl-5'-[2(RS)-(2,4-dichlorophenoxy)propylamino]uridine.

**13.** The use of a compound according to claims 1-12 for the manufacture of medicaments for the control or prophylaxis of viral infections.

**14.** A process for the manufacture of pharmaceutical preparations, characterized by bringing a compound of formula I into a galenical administration form.

**Revendications**

**Revendications pour les Etats contractants suivants : BE, CH, DE, FR, GB, IT, LI, LU, NL, SE**

**1.** Composés de formule générale :

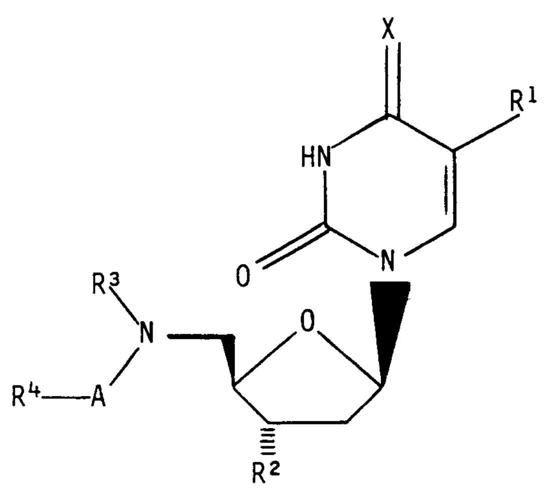

I

dans laquelle A représente un groupe alkylène en $C_1$-$C_8$, $R^1$ représente un halogène, un groupe alkyle en $C_1$-$C_4$ ou halogénoalkyle en $C_1$-$C_4$, $R^2$ représente l'hydrogène, un groupe hydroxy ou acyloxy, $R^3$ représente l'hydrogène ou un groupe alkyle en $C_1$-$C_4$, $R^4$ représente un groupe aryle ou aryloxy et X

représente l'oxygène ou le groupe NH, le groupe acyloxy dérivant d'un acide carboxylique aliphatique, cycloaliphatique, araliphatique ou aromatique, et le groupe aryle étant un groupe phényle portant le cas échéant un ou plusieurs substituants choisis parmi les halogènes, les groupes OH, alkyle en $C_1$-$C_4$, alcoxy en $C_1$-$C_4$, $CF_3$, $NO_2$ et $C_6H_5$,
et leurs tautomères.

**2.** Composés selon la revendication 1, dans lesquels $R^4$ représente un groupe aryle.

**3.** Composés selon la revendication 1 ou 2, dans lesquels A représente un groupe alkylène en $C_1$-$C_4$.

**4.** Composés selon les revendications 1, 2 ou 3, dans lesquels $R^1$ représente un groupe alkyle en $C_1$-$C_4$.

**5.** Composés selon l'une des revendications 1 à 4, dans lesquels $R^2$ représente un groupe hydroxy.

**6.** Composés selon l'une des revendications 1 à 5, dans lesquels $R^3$ représente l'hydrogène ou un groupe méthyle.

**7.** Composés selon l'une des revendications 1 à 6, dans lesquels $R^4$ représente un groupe dihalogéno-phényle.

**8.** Composés selon l'une des revendications 1 à 7, dans lesquels X représente l'oxygène.

**9.** Composés selon l'une des revendications 1 à 8, dans lesquels A représente $-CH_2-$, $-CH_2CH_2-$, $-CH(CH_3)-$ ou $-CH_2-CH(CH_3)-$, $R^1$ représente un groupe éthyle, $R^2$ un groupe hydroxy, $R^3$ l'hydrogène ou un groupe méthyle, $R^4$ un groupe 2,3- ou 2,6-dichlorophényle et X l'oxygène.

**10.** Les composés :
5'-(2,6-dichlorobenzylamino)-2',5'-didésoxy-5-éthyluridine,
5'-[2-(2,6-dichlorophényl)-éthylamino]-2',5'-didésoxy-5-éthyluridine, et
5'-(2,3-dichlorobenzylamino)-2',5'-didésoxy-5-éthyluridine.

**11.** Les composés :
5'-(4-chlorobenzylamino)-5'-désoxy-thymidine,
5'-(2-bromobenzylamino)-2',5'-didésoxy-5-éthyluridine,
5'-[2-(2-bromophényl)-éthylamino]-2',5'-didésoxy-5-éthyluridine,
2',5'-didésoxy-5-éthyl-5'-[2-(2,6-diméthylphényl)-éthylamino]-uridine,
5'-(4-chlorobenzylamino)-2',5'-didésoxy-5-éthyluridine,
2',5'-didésoxy-5-éthyl-5'-[1(R)-phényléthylamino]-uridine,
2',5'-didésoxy-5-éthyl-5'-[1(S)-phényléthylamino]-uridine,
2',5'-didésoxy-5-éthyl-5'-(N-méthylbenzylamino)-uridine,
2',5'-didésoxy-5-éthyl-5'-(phényléthylamino)-uridine,
2',5'-didésoxy-5-éthyl-5'-[2-(2-méthylphényl)-éthylamino]-uridine,
2',5'-didésoxy-5-éthyl-5'-(N-méthylphényléthylamino)-uridine,
5'-[1(S)-benzyléthylamino]-2',5'-didésoxy-5-éthyluridine,
5'-benzylamino-2',5'-didésoxy-5-éthyluridine,
2',5'-didésoxy-5-éthyl-5'-(2-méthylbenzylamino)-uridine, et
5'-benzylamino-5'-désoxythymidine.

**12.** La 2',5'-didésoxy-5-éthyl-5'-[2(RS)-(2,4-dichlorophénoxy)-propylamino]-uridine.

**13.** Les composés des revendications 1 à 12 pour l'utilisation en tant que médicaments, en particulier contre les virus.

**14.** Procédé de préparation des composés de formule générale I et de leurs tautomères, caractérisé en ce que :
a) on fait réagir un composé de formule :

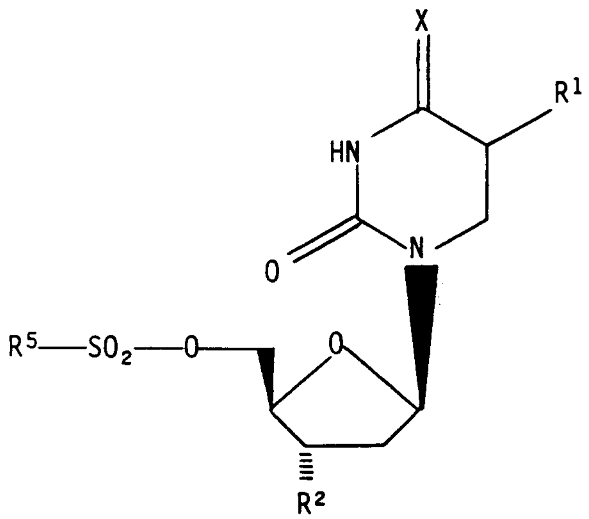

II

dans laquelle $R^1$, $R^2$ et X ont les significations indiquées ci-dessus et $R^5$ représente un groupe alkyle en $C_1$-$C_4$ ou aryle,
ou un tautomère d'un tel composé,
avec un composé de formule :

$R^3$-NH-A-$R^4$ III

dans laquelle A, $R^3$ et $R^4$ ont les significations indiquées ci-dessus, à chaud, ou bien,
b) pour préparer un composé de formule I dans laquelle X représente O ou un tautomère d'un tel composé, on introduit le groupe $R^4$-A-dans un composé de formule :

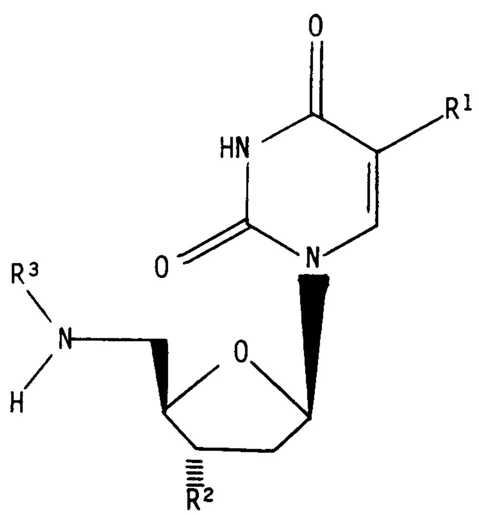

IV

dans laquelle $R^1$, $R^2$ et $R^3$ ont les significations indiquées ci-dessus, ou dans un tautomère d'un tel composé, par alkylation réductive.

**15.** Compositions pharmaceutiques, en particulier à activité antivirale, contenant un composé selon les revendications 1 à 12 et un véhicule pharmaceutique.

**16.** Utilisation d'un composé selon les revendications 1 à 12 pour la préparation de médicaments servant au traitement ou à la prophylaxie d'infections virales.

**Revendications pour les Etats contractants suivants : AT, ES, GR**

**1.** Procédé de préparation des composés de formule générale :

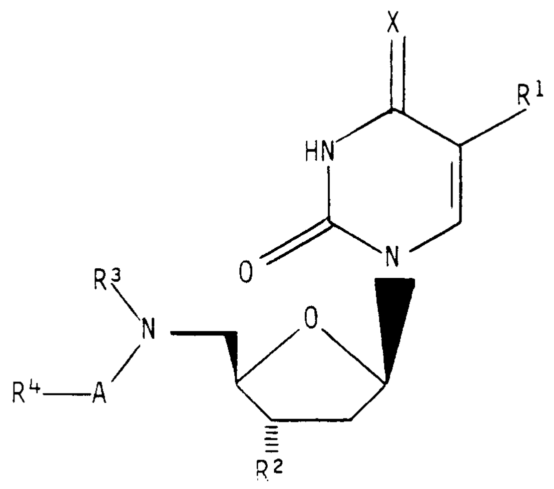

I

dans laquelle A représente un groupe alkylène en $C_1$-$C_8$, $R^1$ représente un halogène, un groupe alkyle en $C_1$-$C_4$ ou halogénoalkyle en $C_1$-$C_4$, $R^2$ représente l'hydrogène, un groupe hydroxy ou acyloxy, $R^3$ représente l'hydrogène ou un groupe alkyle en $C_1$-$C_4$, $R^4$ représente un groupe aryle ou aryloxy et X l'oxygène ou le groupe NH, le groupe acyloxy dérivant d'un acide carboxylique aliphatique, cycloaliphatique, araliphatique ou aromatique, et le groupe aryle consistant en un groupe phényle portant éventuellement un ou plusieurs substituants choisis parmi les halogènes, les groupes OH, alkyle en $C_1$-$C_4$, alcoxy en $C_1$-$C_4$, $CF_3$, $NO_2$ et $C_6H_5$, et de leurs tautomères, caractérisé en ce que

(a) on fait réagir un composé de formule :

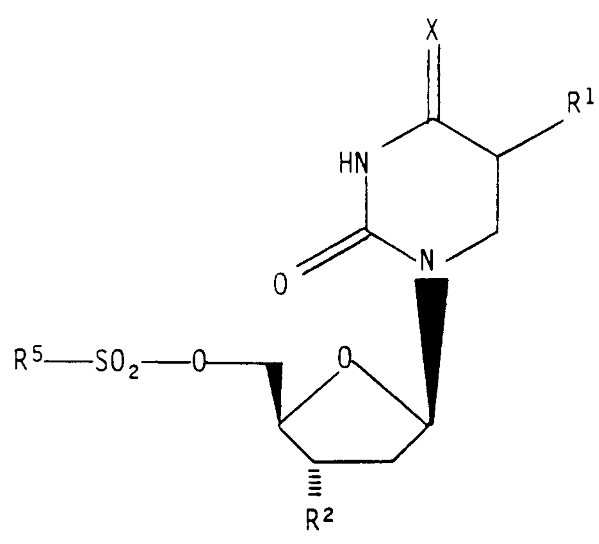

II

dans laquelle $R^1$, $R^2$ et X ont les significations indiquées ci-dessus et $R^5$ représente un groupe alkyle en $C_1$-$C_4$ ou aryle,

ou un tautomère d'un tel composé, avec un composé de formule :

$R^3$-NH-A-$R^4$ III

dans laquelle A, $R^3$ et $R^4$ ont les significations indiquées ci-dessus, à chaud, ou bien

(b) pour préparer un composé de formule I dans laquelle X représente O ou un tautomère d'un tel composé, on introduit le groupe $R^4$-A dans un composé de formule :

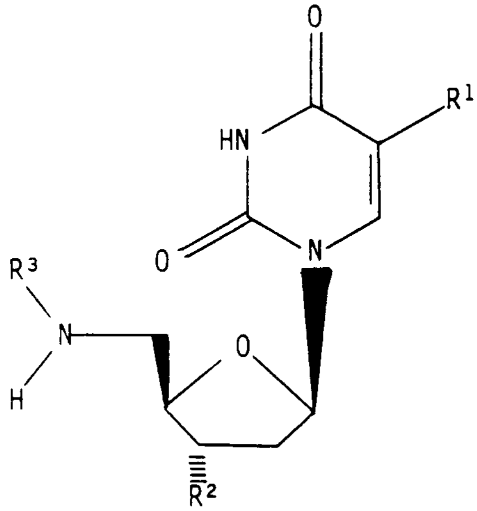

IV

dans laquelle $R^1$, $R^2$ et $R^3$ ont les significations indiquées ci-dessus, ou dans un tautomère d'un tel composé, par alkylation réductive.

**2.** Procédé selon la revendication 1, dans lequel R représente un groupe aryle.

**3.** Procédé selon la revendication 1 ou 2, dans lequel A représente un groupe alkylène en $C_1$-$C_4$.

**4.** Procédé selon les revendications 1, 2 ou 3, dans lequel $R^4$ représente un groupe alkyle en $C_1$-$C_4$.

**5.** Procédé selon l'une des revendications 1 à 4, dans lequel $R^2$ représente un groupe hydroxy.

**6.** Procédé selon l'une des revendications 1 à 5, dans lequel $R^3$ représente l'hydrogène ou un groupe méthyle.

**7.** Procédé selon l'une des revendications 1 à 6, dans lequel $R^4$ représente un groupe dihalogénophényle.

**8.** Procédé selon l'une des revendications 1 à 7, dans lequel X représente l'oxygène.

**9.** Procédé selon l'une des revendications 1 à 8, dans lequel A représente $-CH_2-$, $-CH_2CH_2-$, $-CH(CH_3)-$ ou $-CH_2-CH(CH_3)-$, R représente un groupe éthyle, $R^2$ un groupe hydroxy, $R^3$ l'hydrogène ou un groupe méthyle, $R^4$ un groupe 2,3- ou 2,6-dichlorophényle et X l'oxygène.

**10.** Procédé selon la revendication 1 pour préparer les composés :
5'-(2,6-dichlorobenzylamino)-2',5'-didésoxy-5-éthyluridine,
5'-[2-(2,6-dichlorophényl)-éthylamino]-2',5'-didésoxy-5-éthyluridine, et
5'-(2,3-dichlorobenzylamino)-2',5'-didésoxy-5-éthyluridine.

**11.** Procédé selon la revendication 2 pour préparer les composés :
5'-(4-chlorobenzylamino)-5'-désoxy-thymidine,
5'-(2-bromobenzylamino)-2',5'-didésoxy-5-éthyluridine,
5'-[2-(2-bromophényl)-éthylamino]-2',5'-didésoxy-5-éthyluridine,
2',5'-didésoxy-5-éthyl-5'-[2-(2,6-diméthylphényl)-éthylamino]-uridine,
5'-(4-chlorobenzylamino)-2',5'-didésoxy-5-éthyluridine,
2',5'-didésoxy-5-éthyl-5'-[1(R)-phényléthylamino]-uridine,
2',5'-didésoxy-5-éthyl-5'-[1(S)-phényléthylamino]-uridine,
2',5'-didésoxy-5-éthyl-5'-(N-méthylbenzylamino)-uridine,
2',5'-didésoxy-5-éthyl-5'-(phényléthylamino)-uridine,
2',5'-didésoxy-5-éthyl-5'-[2-(2-méthylphényl)-éthylamino]-uridine,
2',5'-didésoxy-5-éthyl-5'-(N-méthylphényléthylamino)-uridine,
5'-[1(S)-benzyléthylamino]-2',5'-didésoxy-5-éthyluridine,

5'-benzylamino-2',5'-didésoxy-5-éthyluridine,
2',5'-didésoxy-5-éthyl-5'-(2-méthylbenzylamino)-uridine, et
5'-benzylamino-5'-désoxythymidine.

**12.** Procédé selon la revendication 1 pour préparer la 2',5'-didésoxy-5-éthyl-5'-[2(RS)-(2,4-dichlorophénoxy)-propylamino]-uridine.

**13.** Utilisation d'un composé selon les revendications 1 à 12 pour la préparation de médicaments servant eux-mêmes au traitement ou à la prophylaxie d'infections virales.

**14.** Procédé de préparation de compositions pharmaceutiques, caractérisé en ce que l'on met un composé de fomrule I sous une forme d'administration galénique.